# EUROPEAN PATENT APPLICATION

(11) **EP 3 461 887 A1**
(43) Date of publication of application: **03.04.2019**
(21) Application number: 17763586.9
(22) Date of filing: 09.03.2017
(51) Int. Cl.: C12N 5/0775, A61K 35/28

(54) **COMPOSITION FOR PROLIFERATION, DIFFERENTIATION PROMOTION, OR SENESCENCE INHIBITION OF STEM CELLS, CONTAINING JAK1 INHIBITOR AS ACTIVE INGREDIENT**

(30) Priority: 09.03.2016 KR 20160028130
(71) Applicant: University Of Ulsan Foundation For Industry Cooperation, Ulsan 44610 (KR)
(72) Inventor: KIM, Seong-Who, Seoul 05646 (KR); KIM, Sang-Yoon, Seoul 05553 (KR); LEE, Hyang-Ju, Sejong-si 30063 (KR)
(74) Representative: Roos, Peter
(86) International application number: PCT/KR2017/002552
(87) International publication number: WO 2017/155325

(57) **Abstract**

The present invention relates to a composition for proliferation, differentiation promotion, and senescence inhibition of stem cells, comprising a JAK1 inhibitor as an active ingredient. JAK1 inhibitor-treated stem cells or JAK1 knockout stem cells, of the present invention, have an activity for decreasing senescence and increasing the survival rate of the stem cells, thereby being usable in a composition for proliferation, differentiation promotion, and senescence inhibition of stem cells. In addition, JAK1 knockout stem cells have an activity for inhibiting or decreasing the expression of HLA-A or HLA-B, which is an immunogenicity related protein, and promoting or increasing the expression of TGF-b or HGF, which is a T cell activity inhibitory protein, thereby also being usable in the prevention or treatment of immune diseases.

SEQUENCE LISTING

<110>  University of Ulsan Foundation For Industry Cooperation

<120>  Composition for promoting proliferation, differentiation or
       anti-aging of stem cell using Janus kinase 1 inhibitor

<130>  PN1601-001

<160>  3

<170>  PatentIn version 3.2

<210>  1
<211>  3426
<212>  DNA
<213>  JAK1 cDNA sequence

<400>  1
atggctttct gtgctaaaat gaggagctcc aagaagactg aggtgaacct ggaggcccct      60

gagccagggg tggaagtgat cttctatctg tcggacaggg agcccctccg gctgggcagt     120

ggagagtaca cagcagagga actgtgcatc agggctgcac aggcatgccg tatctctcct     180

ctttgtcaca acctctttgc cctgtatgac gagaacacca agctctggta tgctccaaat     240

cgcaccatca ccgttgatga caagatgtcc ctccggctcc actaccggat gaggttctat     300

ttcaccaatt ggcatggaac caacgacaat gagcagtcag tgtggcgtca ttctccaaag     360

aagcagaaaa atggctacga gaaaaaaaag attccagatg caacccctct ccttgatgcc     420

agctcactgg agtatctgtt tgctcaggga cagtatgatt tggtgaaatg cctggctcct     480

attcgagacc ccaagaccga gcaggatgga catgatattg agaacgagtg tctagggatg     540

gctgtcctgg ccatctcaca ctatgccatg atgaagaaga tgcagttgcc agaactgccc     600

aaggacatca gctacaagcg atatattcca gaaacattga ataagtccat cagacagagg     660

aaccttctca ccaggatgcg gataaataat gttttcaagg atttcctaaa ggaatttaac     720

aacaagacca tttgtgacag cagcgtgtcc acgcatgacc tgaaggtgaa atacttggct     780

accttggaaa ctttgacaaa acattacggt gctgaaatat ttgagacttc catgttactg     840

atttcatcag aaaatgagat gaattggttt cattcgaatg acggtggaaa cgttctctac     900

tacgaagtga tggtgactgg gaatcttgga atccagtgga ggcataaacc aaatgttgtt     960

tctgttgaaa aggaaaaaaa taaactgaag cggaaaaaac tggaaaataa acacaagaag    1020

gatgaggaga aaaacaagat ccgggaagag tggaacaatt tttcttactt ccctgaaatc    1080

actcacattg taataaagga gtctgtggtc agcattaaca agcaggacaa caagaaaatg    1140

gaactgaagc tctcttccca cgaggaggcc ttgtcctttg tgtccctggt agatggctac    1200

ttccggctca cagcagatgc ccatcattac ctctgcaccg acgtggcccc cccgttgatc    1260

gtccacaaca tacagaatgg ctgtcatggt ccaatctgta cagaatacgc catcaataaa    1320

ttgcggcaag aaggaagcga ggaggggatg tccgtgctga ggtggagctg caccgacttt 1380
gacaacatcc tcatgaccgt cacctgcttt gagaagtctg agcaggtgca gggtgcccag 1440
aagcagttca agaactttca gatcgaggtg cagaagggcc gctacagtct gcacggttcg 1500
gaccgcagct tccccagctt gggagacctc atgagccacc tcaagaagca gatcctgcgc 1560
acggataaca tcagcttcat gctaaaacgc tgctgccagc ccaagccccg agaaatctcc 1620
aacctgctgg tggctactaa gaaagcccag gagtggcagc ccgtctaccc catgagccag 1680
ctgagtttcg atcggatcct caagaaggat ctggtgcagg gcgagcacct tgggagaggc 1740
acgagaacac acatctattc tgggaccctg atggattaca aggatgacga aggaacttct 1800
gaagagaaga agataaaagt gatcctcaaa gtcttagacc ccagccacag ggatatttcc 1860
ctggccttct tcgaggcagc cagcatgatt agacaggtct cccacaaaca catcgtgtac 1920
ctctatggcg tctgtgtccg cgacgtggag aatatcatgg tggaagagtt tgtggaaggg 1980
ggtcctctgg atctcttcat gcaccggaaa agcgatgtcc ttaccacacc atggaaattc 2040
aaagttgcca aacagctggc cagtgccctg agctacttgg aggataaaga cctggtccat 2100
ggaaatgtgt gtactaaaaa cctcctcctg gcccgtgagg gaatcgacag tgagtgtggc 2160
ccattcatca agctcagtga ccccggcatc cccattacgg tgctgtctag gcaagaatgc 2220
attgaacgaa tcccatggat tgctcctgag tgtgttgagg actccaagaa cctgtgtgtg 2280
gctgctgaca agtggagctt tggaaccacg ctctgggaaa tctgctacaa tggcgagatc 2340
cccttgaaag acaagacgct gattgagaaa gagagattct atgaaagccg gtgcaggcca 2400
gtgacaccat catgtaagga gctggctgac ctcatgaccc gctgcatgaa ctatgacccc 2460
aatcagaggc ctttcttccg agccatcatg agagacatta ataagcttga agagcagaat 2520
ccagatattg tttcagaaaa aaaaccagca actgaagtgg accccacaca ttttgaaaag 2580
cgcttcctaa agaggatccg tgacttggaa gagggacact ttgggaaggt tgagctctgc 2640
aggtatgacc ccgaagacaa tacaggggag caggtggctg ttaaatctct gaagcctgag 2700
agtggaggta accacatagc tgatctgaaa aaggaaatcg agatcttaag gaacctctat 2760
catgagaaca ttgtgaagta caaaggaatc tgcacagaag acggaggaaa tggtattaag 2820
ctcatcatgg aatttctgcc ttcgggaagc cttaaggaat atcttccaaa gaataagaac 2880
aaaataaacc tcaaacagca gctaaaatat gccgttcaga tttgtaaggg gatggactat 2940
ttgggttctc ggcaatacgt tcaccgggac ttggcagcaa gaaatgtcct tgttgagagt 3000
gaacaccaag tgaaaattgg agacttcggt ttaaccaaag caattgagac cgataaggag 3060
tattacaccg tcaaggatga ccgggacagc ccaaagtttg ggtatgctcc agaatgttta 3120
atgcaatcta aattttatat tgcctctgac gtctggtctt ttggagtcac tctgcatgag 3180
ctgctgactt actgtgattc agattctagt cccatggctt tgttcctgaa aatgataggc 3240

ccaacccatg gccagatgac agtcacaaga cttgtgaata cgttaaaaga aggaaaacgc 3300

ctgccgtgcc cacctaactg tccagatgag gtttatcagc ttatgagaaa atgctgggaa 3360

ttccaaccat ccaatcggac aagctttcag aaccttattg aaggatttga agcactttta 3420

aaataa 3426

<210> 2
<211> 1141
<212> PRT
<213> JAK1 amino acid sequence

<400> 2

<210>  3
<211>  20
<212>  DNA
<213>  JAK1 sgRNA sequence

<400>  3
atgacgagaa caccaagctc                                                20

## Description

### [Technical Field]

The present invention relates to a composition for proliferation, differentiation promotion or senescence inhibition of stem cells comprising a JAK1 inhibitor as an active ingredient.

### [Background Art]

Stem cells are multipotent stem cells derived from various adult cells such as bone marrow, umbilical cord blood, placenta (or placental tissue cells), and fat (or adipose tissue cells). For example, various researches have been conducted to develop mesenchymal stem cells derived from bone marrow as a cell therapeutic agent by their multipotency capable of being differentiated into fatty tissue, bone/cartilage tissue and muscle tissue. Meanwhile, it is known that similar to other human primary cultured cells, the cell division of mesenchymal stem cells are rapidly reduced in *in vitro* culture due to senescence mechanism independent of telomere shortening.

Although the mechanism of such senescence has not yet been identified, p16 (INK4a), a Cdk inhibitory protein, is expressed and accumulated by the accumulation of environmental stress due to prolonged in vitro culture and the inhibition of the activity of the Cdk protein responsible for cell growth has been regarded as a major cause. When Bmi-1 oncogene is expressed in mesenchymal stem cells and the expression of p16 is inhibited, it has been shown that cellular senescence is inhibited. In addition, it has been reported that when the mRNA expression of p21 (Cip1), p53, and p16 (INK4a) is inhibited by treating FGF-2 during culture, growth-arrested mesenchymal stem cells in the G1 phase is inhibited. Furthermore, Korean Patent Publication No. 10-2009-0108141 discloses a method of inhibiting senescence of mesenchymal stem cells by transforming a gene encoding Wip1 protein into mesenchymal stem cells.

However, since Bmi-1 itself is an oncogene that can cause tumors, the possibility of tumor formation cannot be excluded when mesenchymal stem cells treated with Bmi-1 and hTERT (telemorase catalytic subunit) are used as cell therapeutic agents. In addition, the method of treating FGF-2 during the culture expects not only the increase in cost due to continuous use of the relatively expensive FGF-2 during the culture, but also the effect of temporary increase in cell growth rather than inhibition of senescence owing to FGF-2 and there is a limit to inhibiting senescence.

In addition, there have been attempts to treat diseases using various adult stem cell therapeutic agents in the past, but due to the senescence of inflammatory stem cells, there are problems of stem cell survival rate after transplantation and thus it has been difficult to secure the stem cell treatment effect.

To solve the above problems, the present inventors developed cell therapeutic agent using stem cells capable of enhancing the therapeutic effect by proliferation, differentiation promotion and senescence inhibition of stem cells, and confirmed that stem cells inhibiting the expression or activity of JAK1 can be used as an excellent cell therapeutic agent for immune diseases, thereby completing the present invention.

### [Disclosure]

### [Technical Problem]

Therefore, an object of the present invention is to provide composition for proliferation, differentiation promotion or senescence inhibition of stem cells.

Also, another object of the present invention is to provide a method of promoting proliferation and differentiation of stem cells, comprising inhibiting an expression or activity of JAK1 (Janus kinase 1) in stem cells.

In addition, another object of the present invention is to provide a method of inhibiting senescence of a stem cell comprising treating a JAK1 (Janus kinase 1) inhibitor or knocking out a JAK1 (Janus kinase 1) in a stem cell.

Furthermore, another object of the present invention is to provide a method of preparing stem cells for cell therapy, comprising treating a JAK1 (Janus kinase 1) inhibitor or knocking out a JAK1 (Janus kinase 1) in a stem cell for cell transplantation.

In addition, another object of the present invention is to provide a stem cell which the expression or activity of JAK1 is inhibited by treating the JAK1 inhibitor, or JAK1 knockout stem cell.

Furthermore another object of the present invention is to provide a cell therapeutic composition for preventing or treating immune diseases, comprising the stem cell which the expression or activity of JAK1 is inhibited as an active ingredient.

In addition, another object of the present invention is to provide a cell therapeutic composition or composition for preventing or treating immune diseases comprising JAK1 inhibitor and stem cells.

In addition, another object of the present invention is to provide a method of preventing or treating immune diseases by administering JAK1 inhibitor and stem cells to a subject.

### [Technical Solution]

In order to achieve the above object, the present invention provides a composition for proliferation, differentiation promotion or senescence inhibition of stem cells comprising a JAK1 (Janus kinase 1) inhibitor as an active ingredient.

In one embodiment of the present invention, the JAK1 inhibitor may be selected from the group consisting of tofacitinib, baricitinib, ruxolitinib and filgotinib.

In one embodiment of the present invention, the JAK1 inhibitor may inhibit or reduce an expression of HLA-A or HLA-B which is an immunogenicity-related protein, and may promote or increase an expression of TGF-b or HGF which inhibits activity of T cells.

In one embodiment of the present invention, the JAK1 inhibitor may reduce the senescence of stem cells and promotes survival rate of stem cells.

In addition, the present invention provides a method of promoting proliferation and differentiation of stem cells, comprising inhibiting an expression or activity of JAK1 (Janus kinase 1) in stem cells.

In one embodiment of the present invention, the expression or activity of JAK1 (Janus kinase 1) may be inhibited by treating a JAK1 (Janus kinase 1) inhibitor or knocking out a JAK1 (Janus kinase 1) gene in a stem cell.

In one embodiment of the present invention, the JAK1 inhibitor may be selected from the group consisting of tofacitinib, baricitinib, ruxolitinib and filgotinib.

In addition, the present invention provides a method of inhibiting senescence of a stem cell comprising: treating a JAK1 (Janus kinase 1) inhibitor or knocking out a JAK1 (Janus kinase 1) in a stem cell.

In one embodiment of the present invention, the JAK1 inhibitor may be selected from the group consisting of tofacitinib, baricitinib, ruxolitinib and filgotinib.

In addition, in the present invention, the method of removing JAK1 may be use the CRISPR-Cas9 gene scissors system, but is not limited thereto.

The present invention also provides a method of preparing stem cells for cell therapy, comprising treating a JAK1 (Janus kinase 1) inhibitor or knocking out a JAK1 (Janus kinase 1) in a stem cell for cell transplantation, wherein the stem cells have followings:
(a) reduced inflammatory senescence of stem cells;
(b) inhibited cell survival rate reduction of stem cells;
(c) increased cell proliferation of stem cells;
(d) increased stemness of stem cells;
(e) decreased immunogenicity of stem cells; and
(f) increased immunomodulatory activity of stem cells.

Furthermore, the present invention provides a cell therapeutic composition for preventing or treating immune diseases, comprising the stem cell prepared by the method of the present invention as an active ingredient.

In one embodiment of the present invention, the immunological disease may be selected from the group consisting of graft-versus-host disease, autoimmune disease, rheumatoid arthritis, Lupus disease, Behcet's disease and Sjogren's disease.

In addition, the present invention also provides a kit or composition for treating an immune disease comprising a JAK1 inhibitor and stem cells.

In addition, the present invention provides a method of preventing or treating immune diseases by administering JAK1 inhibitor and stem cells to a subject.

### [Advantageous Effects]

A stem cell treated with the JAK1 inhibitor or a stem cell in which JAK-1 is knocked out has the activity of decreasing senescence and increasing the survival rate of stem cells and thus has an effect of using a composition for proliferation, differentiation promotion, or senescence inhibition of a stem cell. In addition, the stem cell in which JAK1 is knocked out has an activity of inhibiting or reducing the expression of HLA-A or HLA-B which is an immunogenicity-related protein, and promotes or increases an expression of TGF-b or HGF which inhibits activity of T cells, and is effective for preventing or treating immune diseases.

### [Description of Drawings]

FIG. 1 shows the results of the inflammatory senescence resistance of stem cells by the treatment with JAK1 inhibitor, tofacitinib.
FIG. 2 shows the results of the inflammatory senescence resistance of stem cells by the treatment with the JAK1 inhibitors, ruxolitinib and filgotinib.
FIG 3 shows the results of the inflammatory senescence resistance in JAK1 knock-out stem cells.

### [Best Mode]

The present invention provides a composition for proliferation, differentiation promotion or senescence inhibition of stem cells comprising a JAK1 (Janus kinase 1) inhibitor as an active ingredient.

The JAK1 (Janus kinase 1) gene is preferred to have the nucleic acid sequence represented by SEQ ID NO: 1 and the JAK1 protein preferably has the amino acid sequence represented by SEQ ID NO: 2, but they are not limited thereto.

In the present invention, the JAK1 (Janus kinase 1) inhibitor may be any one selected from the group consisting of an antisense nucleotide complementary to mRNA of the JAK1 gene, a sgRNA (single guide), a shRNA (small hairpin RNA), a siRNA (small interfering RNA) and a ribozyme, and most preferably, it may be one in which the expression of the JAK1 gene is inhibited using the sgRNA of SEQ ID NO: 3.

In addition, according to the present invention, it was confirmed that it has the effect of not only promoting the proliferation and differentiation but also inhibiting senescence in stem cells in which JAK1 is knocked out or treated with JAK1 inhibitor.

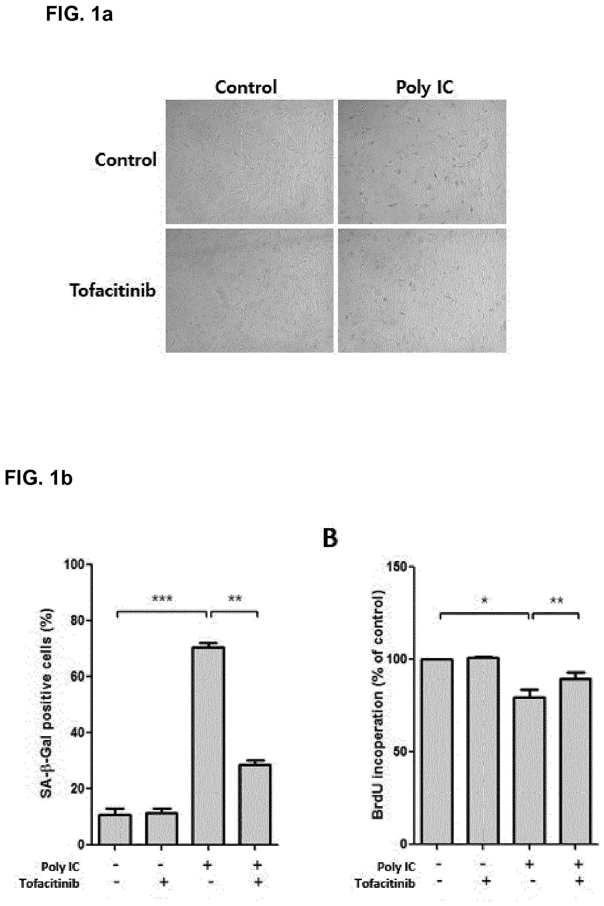

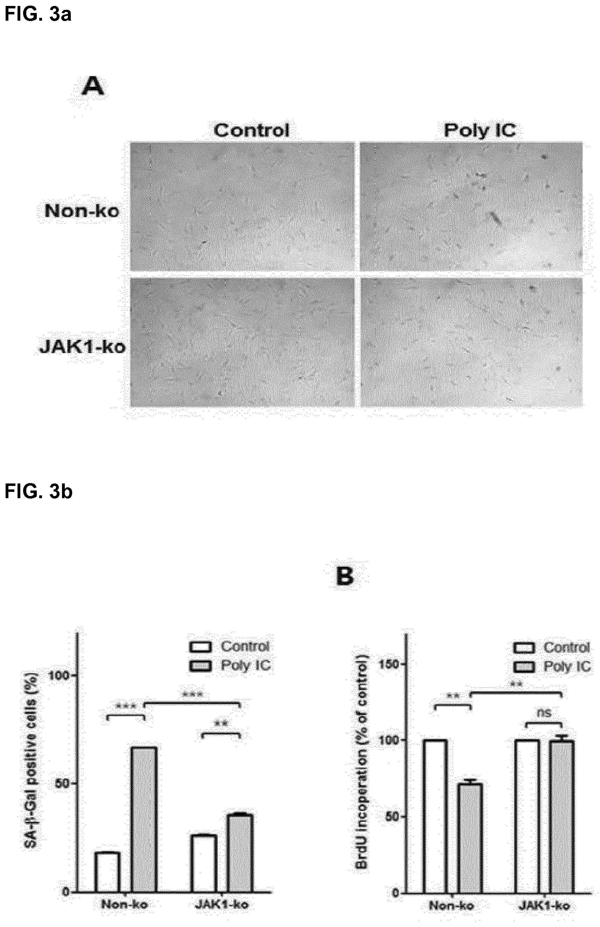

More specifically, in order to confirm whether there is an effect of reducing the senescence of the stem cell according to the treatment of JAK1 inhibitor tofacitinib or the JAK1 knock-out, it was confirmed that the inflammatory senescence induced by poly IC in the stem cells was markedly decreased by the treatment of tofacitinib or the JAK1 knock-out through SA-b-gal staining which is a typical technique of measuring the cellular senescence (FIG. 1A and FIG. 3A).

According to one embodiment of the present invention, in order to confirm the effect of inhibiting the decrease of the survival rate of stem cells after transplantation according to the treatment of JAK1 inhibitor tofacitinib or the JAK1 knock-out, it was confirmed that the cell survival rate by cellular senescence was less reduced in the stem cells induced to poly IC by the treatment of tofacitinib or the JAK1 knock-out through BrdU assay (FIG. 1B and FIG. 3B).

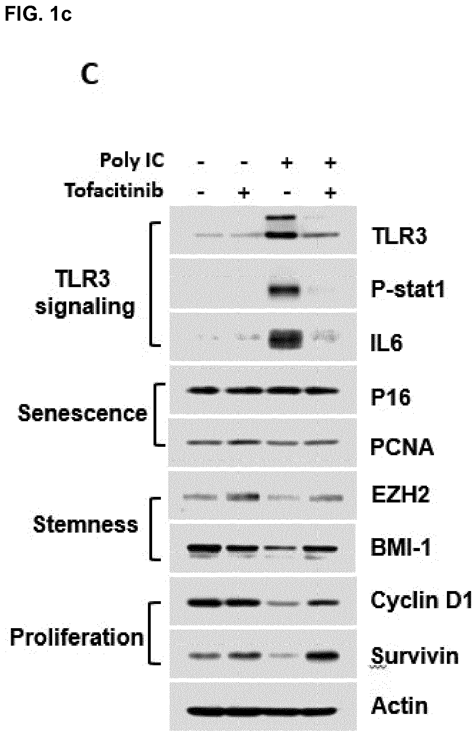

According to another embodiment of the present invention, western blotting was used to confirm the expression of proteins involved in stem cell proliferation, differentiation promotion, stemness and senescenece by treatment of tofacitinib, ruxolitinib and filgotinib or JAK1 knock-out. As a result, the decrease in the expression of the protein associated with senescence (PCNA), stemness (EZH2, BMI-1) and proliferation (survivin, CyclinD1) were less decreased. In addition, the increase in expression of TLR3 signaling-related proteins (TLR3, IL6) were less increased. (FIG. 1C, FIG. 2 and FIG. 3C).

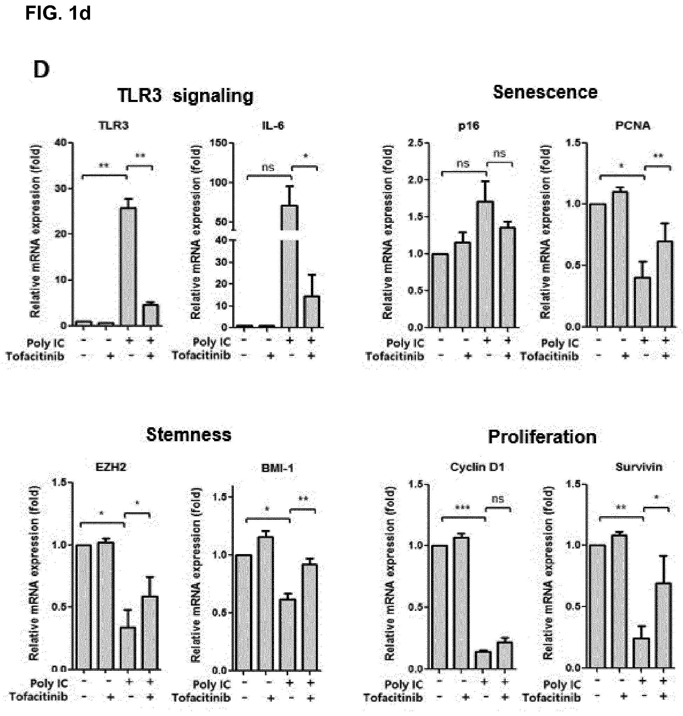

According to another embodiment of the present invention, real time PCR was used to confirm the mRNA expression of proteins involved in stem cell proliferation, differentiation promotion, stemness and senescenece by treatment of tofacitinib, ruxolitinib and filgotinib or JAK1 knock-out. As a result, the decrease in the mRNA expression of the protein associated with senescence (PCNA), stemness (EZH2, BMI-1) and proliferation (survivin, CyclinD1) were less decreased. In addition, the increase in expression of TLR3 signaling-related proteins (TLR3, IL6) were less increased. (FIG. 1D).

Accordingly, the present invention provides a composition for proliferation, differentiation promotion or senescence inhibition of stem cells comprising a JAK1 (Janus kinase 1) inhibitor as an active ingredient.

The term 'stem cell' of the present invention is not limited as long as it has a property of multipotency and may be derived from adult cells such as all known tissues and cells derived from a mammal including a human, preferably a human, and for example, from bone marrow, umbilical cord blood, placenta (or placental tissue cells), fat (or adipose tissue cells), and the like. Preferably, the stem cells may be mesenchymal stem cells derived from human bone marrow, placenta or umbilical cord blood.

In addition, the JAK1 (Janus kinase 1) inhibitor of the present invention may be selected from the group consisting of tofacitinib, baricitinib, ruxolitinib and filgotinib and even though it is not limited thereto, most preferably tofacitinib, ruxolitinib or filgotinib can be used.

As used herein, the term "senescence of stem cells" refers to phenomenon in which the cell growth of the mesenchymal stem cells is stopped or significantly delayed against various stresses from the inside or the outside (for example, oxygen conditions at high concentration during continuous subculture and in vitro culture) and includes cell growth arrest or delay irrespective of telomere shortening of mesenchymal stem cells.

In addition, the present invention provides a method of promoting proliferation and differentiation of stem cells, comprising inhibiting an expression or activity of JAK1 (Janus kinase 1) in stem cells.

The method of promoting the proliferation and differentiation of stem cells according to the present invention comprises treating a JAK1 (Janus kinase 1) inhibitor or knocking out a JAK1 (Janus kinase 1) gene in a stem cell.

The method of inhibiting stem cell senescence according to the present invention provides a method of inhibiting stem cell senescence comprising treating a stem cell with a JAK1 (Janus kinase 1) inhibitor or JAK1 (Janus kinase 1).

The present invention provides a method of inhibiting senescence of a stem cell comprising treating a JAK1 (Janus kinase 1) inhibitor or knocking out a JAK1 (Janus kinase 1) in a stem cell.

The method of inhibiting senescence of a stem cell according to the present invention provides a method of inhibiting senescence of a stem cell comprising treating a stem cell with a JAK1 (Janus kinase 1) inhibitor or JAK1 (Janus kinase 1).

Also, the present invention also provides a method of preparing stem cells for cell therapy, comprising treating a JAK1 (Janus kinase 1) inhibitor or knocking out a JAK1 (Janus kinase 1) in a stem cell for cell transplantation, wherein the stem cells have the following features:
(a) reduced inflammatory senescence of stem cells;
(b) inhibited cell survival rate reduction of stem cells;
(c) increased cell proliferation of stem cells;
(d) increased stemness of stem cells;
(e) decreased immunogenicity of stem cells; and
(f) increased immunomodulatory activity of stem cells.

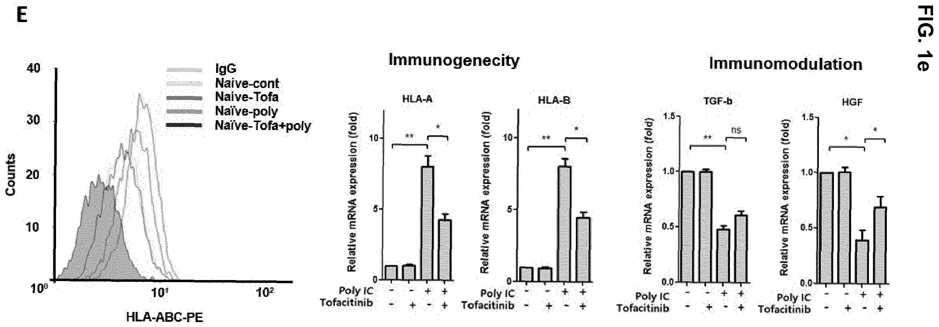

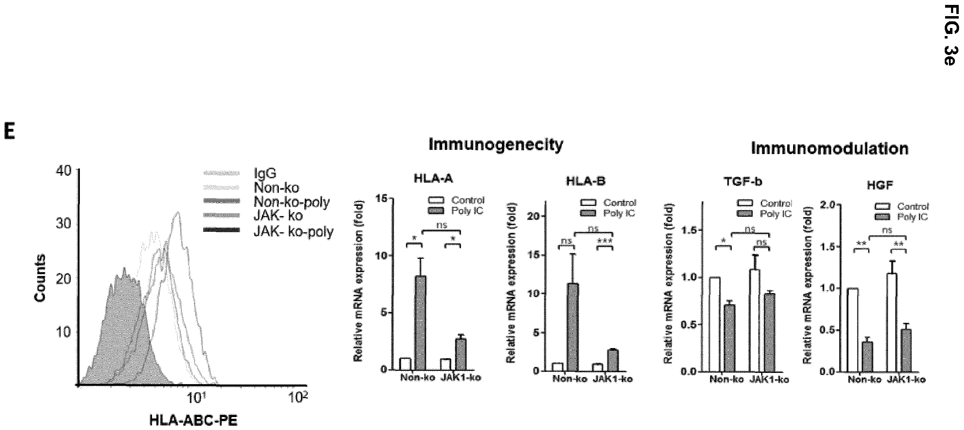

According to the present invention, real time PCR was performed to confirm the expression levels of HLA-A and HLA-B which are immunogenicity-related proteins in stem cells which JAK1 inhibitor, tofacitinib was treated or JAK1 was knocked out and as a result, it was confirmed that the immunogenicity-related proteins HLA-A and HLA-B were increased by the treatment of poly IC, and this increase was less increased by the treatment of tofacitinib or the JAK1 knock-out (FIG. 1E and FIG. 3E).

In another embodiment of the present invention, real time PCR was performed to confirm the expression level of TGF-b and HGF, which are proteins that inhibit T cell activity in stem cells which JAK1 inhibitor, tofacitinib was treated or JAK1 was knocked out and as a result, it was confirmed that the proteins TGF-b and HGF, which inhibit T cell activity were decreased by the treatment of poly IC, and this decrease is less decreased by the treatment of tofacitinib or the JAK1 knock-out (FIG. 1E and FIG. 3E).

Accordingly, as a result, it was confirmed that stem cells in which JAK1 is knocked out have an effect of reducing the immunogenicity of stem cells and increasing immunomodulatory ability.

Therefore, stem cells which JAK1 (Janus kinase 1) inhibitor is treated or is knocked out can be usefully used as a cell therapeutic composition for preventing or treating immune diseases.

In addition, the JAK1 inhibitor and the stem cell of the present invention may be administered jointly to a subject having a disease, and the stem cell may be a stem cell which the JAK1 inhibitor is not pretreated or JAK1 is not knocked out.

Accordingly, the present invention can provide a cell therapy kit or composition for preventing or treating immune diseases comprising JAK1 inhibitors and stem cells.

In the present invention, the 'immune diseases' may be selected from the group consisting of graft-versus-host disease, autoimmune disease, rheumatoid arthritis, Lupus disease, Behcet's disease and Sjogren's disease.

The administration route of the cell therapeutic composition of the present invention can be administered through any conventional route so long as it can reach the target tissue. A parenteral administration, for example, intraperitoneal administration, intravenous administration, intramuscular administration, subcutaneous administration, intradermal administration can be used, but it is not limited thereto.

The composition may be formulated in a suitable form together with a pharmaceutical carrier commonly used in cell therapy. "Pharmaceutically acceptable" refers to compositions which are physiologically acceptable and when administered to humans, do not normally cause allergic reactions such as gastrointestinal disorders, dizziness, etc. A pharmaceutically acceptable carrier includes, for example, water, suitable oils, saline, aqueous carriers for parenteral administration such as aqueous glucose and glycols, etc., and may additionally contain stabilizers and preservatives. Suitable stabilizers are antioxidants such as sodium hydrogen sulfite, sodium sulfite or ascorbic acid. Suitable preservatives include benzalkonium chloride, methyl- or propyl-paraben and chlorobutanol. Other pharmaceutically acceptable carriers can be found in Remington's Pharmaceutical Sciences, 19th ed., Mack Publishing Company, Easton, PA, 1995).

The cell therapeutic composition of the present invention may comprise a therapeutically effective amount of a cell therapeutic agent for treating diseases.

The term of therapeutically effective amount refers to the amount of active ingredient or pharmaceutical composition that induces a biological or medical response in a tissue system, animal or human, as contemplated by a researcher, veterinarian, physician or other clinician and it includes an amount that induces the relief of the symptoms of the disease or disorder being treated. It is apparent to those skilled in the art that the cell therapeutic agent contained in the composition of the present invention will vary depending on the desired effect. Therefore, the optimal cell therapeutic agent content can be readily determined by those skilled in the art and will vary with kind of disease, severity of disease, amount of other ingredients contained in the composition, type of formulation, and patient's age, weight, general health status, sex and diet, the time of administration, the route of administration and the rate of administration of the composition, the duration of the treatment, the drug being co-administered, and the like. It is important to include all of these factors in an amount that can achieve the maximum effect in a minimal amount without side effects. For example, the composition of the present invention may contain a cell therapeutic agent of 1x10⁴ cells/kg to 1x10⁸ cells/kg.

The present invention also provides the use of a composition comprising a cell therapeutic agent as an active ingredient for the preparation of a medicament for preventing or treating immune diseases. The composition of the present invention comprising the cell therapeutic agent as an active ingredient can be used for the preparation of a medicament for preventing or treating immune diseases.

In addition, the present invention provides a method of preventing or treating immune diseases comprising administering to a mammal a therapeutically effective amount of a cell therapeutic agent. In addition, stem cells treated with JAK1 inhibitor or JAK1 is knocked out can be used as a cell therapeutic agent, and stem cells can be co-administered with a JAK1 inhibitor.

As used herein, the term 'mammal' refers to a mammal that is the subject of treatment, observation or experimentation, preferably a human.

In the treatment method of the present invention, in the case of an adult, when the cell therapeutic composition of the present invention is administered once to several times a day, the cell therapeutic agent contained in the composition is preferably included in amount of 1x10⁴ cells/kg to 1x10⁸.

In the treatment method of the present invention, the composition comprising the cell treatment agent of the present invention as an active ingredient can be administered by any conventional route such as rectal, intravenous, intraarterial, intraperitoneal, intramuscular, intrasternal, transdermal, topical, intraocular or intradermal route.

Hereinafter, the present invention will be described in detail with reference to the following examples. It should be noted, however, that the following examples are illustrative of the present invention and are not intended to limit the scope of the present invention.

### <Example 1>

### Confirmation of resistance to inflammatory senescence according to treatment with JAK1 inhibitor, tofacitinib

### <1-1> Confirmation of reduction of stem cell senescence by tofacitinib treatment

The present inventors used SA-b-gal staining which is a representative cellular senescence measurement technique, in order to confirm whether there is an effect of decreasing stem cell senescence by tofacitinib treatment.

More specifically, stem cells were plated in 6 wells (2×10⁴ cells/well) and treated with poly IC (1 ug/ml) one day later. Tofacitinib (1 ug/ml) was pretreated 2 hours before the treatment of poly IC. After 2 days of the poly IC treatment, the degree of senescence was measured using senescence cells histochemical staining kit (CS0030, Sigma). For this, the plate was washed twice with PBS and fixed with fixation buffer (2% formaldehyde, 0.2% glutaraldehyde, 7.04 mM Na₂HPO₄, 1.47 mM KH₂PO₄, 0.137 M NaCl, and 2.68 mM KCl) for 6 minutes and reacted with staining buffer (6 mM Potassium Ferricyanide, 6 mM Potassium Ferrocyanide, 1 mg/ml X-gal Solution in phosphate buffer (pH 6)) at 37 °C for 24 hours. The number of b-galactoxidase-positive cells was measured by microscopic observation and the degree of cellular senescence was shown in a graph.

As a result, as shown in FIG. 1A, it was found that inflammatory senescence induced by poly IC in the stem cells was significantly reduced by the pre-treatment with tofacitinib.

### <1-2> Confirmation of inhibition of survival rate reduction of stem cells after transplantation according to tofacitinib treatment

The present inventors used the BrdU assay to observe the effect of inhibiting the decrease of the survival rate of stem cells after transplantation according to tofacitinib treatment.

More specifically, cells were plated in 96 wells (1×10³ cells/well) and treated with poly IC (1 µg/ml) one day later. Tofacitinib (1 ug/ml) was pretreated 2 hours before the treatment of poly IC. After 2 days of poly IC treatment, cell survival rate was measured using BrdU cell proliferation ELISA (11647229001, Roche). For this, the BrdU labeling solution was treated at 10 µl/well for 4 hours at 37 °C. Then, fixdenat buffer was treated by 200ul/well for 30 minutes to immobilize the cells and reacted with 100ul/well of anti BrdU-POD solution for 90 minutes. Thereafter, 100ul of the substrate solution was treated, the OD value at 370nm wavelength was measured by ELISA, and the cell survival rate was plotted.

As a result, as shown in FIG. 1B, it was confirmed that cell survival rate by cellular senescence induced by poly IC in stem cells was less decreased by pre-treatment with tofacitinib.

### <1-3> Confirmation of expression of proteins involved in proliferation and differentiation promotion of stem cell and sternness increase according to tofacitinib treatment using Western blotting

The present inventors have confirmed the expression of proteins involved in proliferation and differentiation promotion of stem cell and stemness increase according to tofacitinib treatment using Western blotting.

More specifically, the stem cells were plated in a 10 cm dish (3x10⁵ cells/dish) and treated with poly IC (1 ug/ml) one day later. Tofacitinib (1 ug/ml) was pretreated 2 hours before poly IC treatment. After 2 days of poly IC treatment, western blotting was performed for analyzing the protein expression.

For this, the cells were treated with RIPA lysis buffer (1% Np-40, 0.5% Nadeoxycholate, 0.4% SDS, 0.15 M NaCl, 0.05 M Tris-HCI (pH 8), Halt Protease and Phosphatase Inhibitor Cocktail (78440, Thermo Scientific)) and reacted in ice for 30 minutes to extract the cells. The cell lysate was centrifuged and the supernatant was collected and quantified using bicinchoninic acid protein assay kit (23225, Thermo Scientific). 10 ug of the protein extract was separated by 12% SDS-PAGE and transferred to a nitrocellulose membrane. After reacting for 1 h in blocking buffer (0.1% Tween 20, 5% BSA in tris buffered saline) at room temperature (RT), the primary antibody was treated and reacted overnight at 4 °C. The primary antibodies used were TLR3 (#6961, Cell Signaling), phospho-stat1 (#7649, Cell signaling), IL-6 (ab6672, abcam), p16 (ab51243, abcam), PCNA (610665, BD Bioscience), EZH2 (ab3748, abcam), BMI1 (39993, Active Motif), cyclinD1 (abcam, ab134175), survivin (GTX100052, Genetex) and b-actin (A5441, Sigma). After washing three times with TBST solution, peroxidase-conjugated secondary antibody was reacted for 1 hour at RT and protein bands were analyzed using C-DiGit Blot Scanner (LI-COR).

As a result, the expression of proteins associated with senescence (PCNA), stemness (EZH2, BMI-1), proliferation (survivin, CyclinD1) related proteins was less decreased by the pre-treatment with tofacitinib. (FIG. 1C).

### <1-4> Confirmation of expression of proteins involved in proliferation and differentiation promotion of stem cell and sternness increase according to tofacitinib treatment using real time PCR

The present inventors have confirmed the expression of proteins involved in proliferation and differentiation promotion of stem cell and stemness increase according to tofacitinib treatment using real time PCR.

For this, cells were treated with TRIzol reagent to lyse the cells, and treated with chloroform to separate the aqueous solution layer containing the RNA. The separated aqueous solution was treated with isopropanol to precipitate RNA and centrifuged to extract RNA only. The extracted RNA was quantified using nanodrop. 2 ug of RNA was synthesized using TOPscript cDNA synthesis kit (RT200, Enzynomics) as a cDNA mixture and used as a template for real time PCR. Real-time PCR was performed using the CFX Connect Real-Time PCR Detection System (Bio-rad) and primers used were shown in Table 1. Relative mRNA expression levels were analyzed using the comparative threshold cycles (2-^{Ct}) method.

**[Table 1] Primer sequence used in present invention**

| **Target gene** | **primer sequence** | |
|---|---|---|
| TLR3 | Forward | TGGTGAAGGAGAGCTATCCACA |
| | Reverse | TCCCAAGCCTTCAACGACTG |
| IL-6 | Forward | CAGCTCTGGCTTGTTCCTCAC |
| | Reverse | CAATGAGGAGACTTGCCTGGTG |
| p16 | Forward | GGGTCGGGTAGAGGAGGTG |
| | Reverse | GCCTCCGACCGTAACTATTCG |
| PCNA | Forward | GGACATACTGGTGAGGTTCAC |
| | Reverse | CACGTCTCTTTGGTGCAGCTC |
| EZH2 | Forward | GGACCACAGTGTTACCAGCAT |
| | Reverse | GTGGGGTCTTTATCCGCTCAG |
| BMI-1 | Forward | TTCTTTGACCAGAACAGATTGG |
| | Reverse | GCATCACAGTCATTGCTGCT |
| CyclinD1 | Forward | TGGAGCCCGTGAAAAAGAGC |
| | Reverse | TCTCCTTCATCTTAGAGGCCAC |
| Survivin | Forward | CCGGACGAATGCTTTTTATG |
| | Reverse | GCCCAGTGTTTCTTCTGCTT |
| TGF-b | Forward | CAATTCCTGGCGATACCTCAG |
| | Reverse | GCACAACTCCGGTGACATCAA |
| HGF | Forward | GCTATCGGGGTAAAGACCTACA |
| | Reverse | CGTAGCGTACCTCTGGATTGC |
| HLA-A | Forward | AAAAGGAGGGAGTTACACTCAGG |
| | Reverse | GCTGTGAGGGACACATCAGAG |
| HLA-B | Forward | CTACCCTGCGGAGATCA |
| | Reverse | ACAGCCAGGCCAGCAACA |

As a result of the above analysis, the decrease in the expression of the protein associated with senescence (PCNA), stemness (EZH2, BMI-1) and proliferation (survivin, CyclinD1) were less decreased by the pre-treatment with tofacitinib. In addition, the increase in expression of TLR3 signaling-related proteins (TLR3, IL6) were less increased. (FIG. 1D).

### <1-5> Confirmation of reduction of immunogenicity by tofacitinib treatment

The present inventors have confirmed the degree of expression of HLA-A and HLA-B which are immunogenicity-related proteins according to tofacitinib treatment using FACS analysis and real time PCR.

More specifically the stem cells were treated with trypsin/EDTA and separated into single cells and mouse anti-human HLA-ABC (555553, BD Biosciences) was reacted at RT for 1 hour and washed three times and then analyzed using a FACS calibur instrument (BD Biosciences).

As a result, it was confirmed that the immunogenicity-related proteins HLA-A and HLA-B were increased by the poly IC treatment, and this increase was less increased by the pre-treatment of tofacitinib (FIG. 1 E).

### <1-6> Confirmation of increase in immunomodulatory ability by tofacitinib treatment

The present inventors have confirmed the expression level of TGF-b and HGF which are proteins that inhibit T cell activity by tofacitinib treatment using real time PCR.

A concrete experimental method of real time PCR is the same as the Example <1-4>.

As a result, it was confirmed that the TGF-b and HGF which are proteins inhibiting T cell activation, were reduced by treatment of poly IC, and this decrease was less decreased by pre-treatment of tofacitinib (FIG. 1E).

### <1-7> Confirmation of expression of proteins involved in proliferation and differentiation promotion of stem cell and sternness increase according to ruxolitinib or filgotinib treatment using Western blotting

The expression of proteins involved in proliferation and differentiation promotion of stem cells and stemness increase was confirmed by Western blotting in the same manner as in Example 1-3 using JAK1 inhibitors ruxolitinib or filgotinib (GLPG0634).

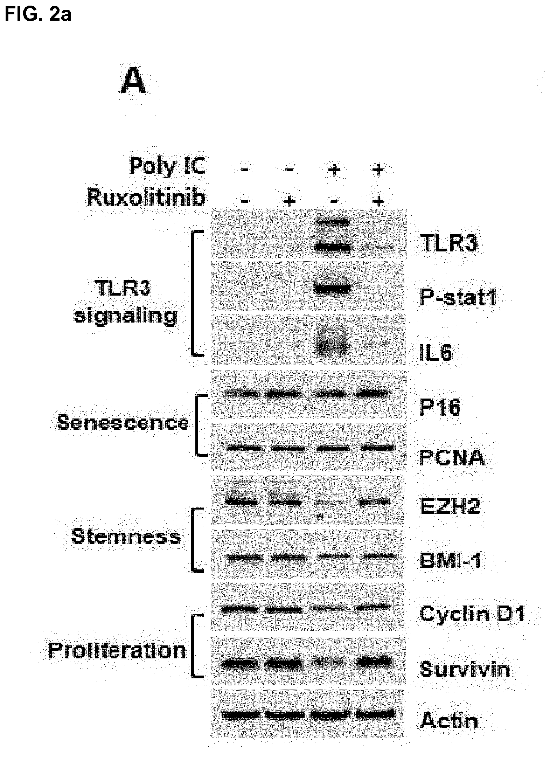

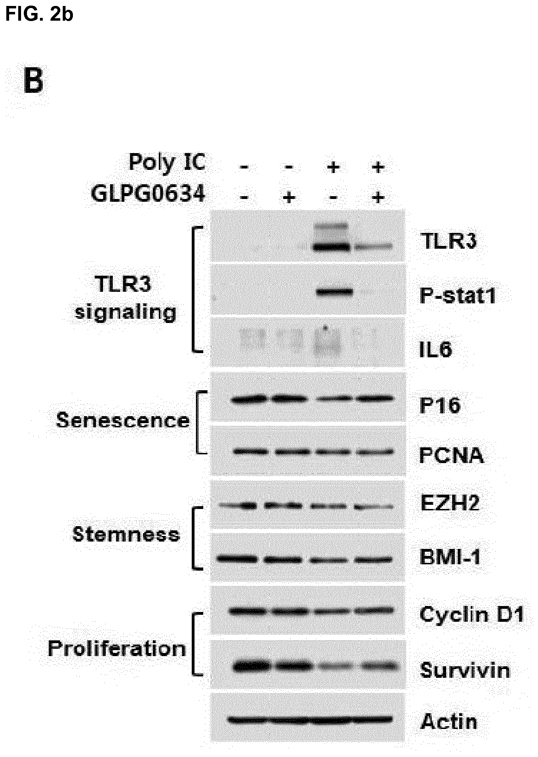

The results are shown in FIG. 2, the decrease in the expression of the protein associated with senescence (PCNA), stemness (EZH2, BMI-1) and proliferation (survivin, CyclinD1) were less decreased according to the treatment of the JAK1 inhibitors. In addition, the increase in expression of TLR3 signaling-related proteins (TLR3, IL6) were less increased.

### <Example 2>

### Confirmation of inflammatory senescence resistance in JAK1 knock-out stem cells

### <2-1> Confirmation of reduction of stem cell senescence by JAK1 knock-out

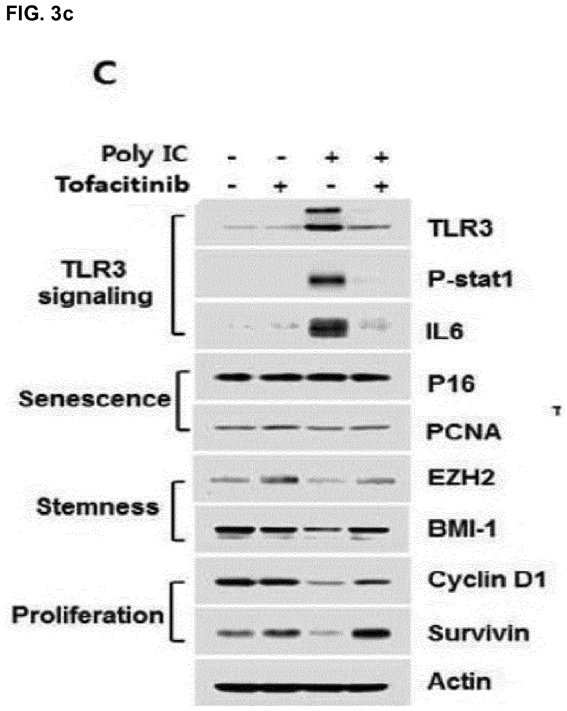

The present inventors cloned the JAK1 gide RNA sequence into the plentiCRISPRv2 vector for the production of JAK1 knock-out cells. PlentiCRISPR-JAK1, pMD2.G and psPAX2 plasmids were then transfected into 293T cells for packaging with lentivirus. Three days after the transfection, stem cells were treated with the culture medium containing the virus to infect plentiCRISPR-JAK1 lentivirus. A puromycin selection was performed using the puromycin resistance gene present in the plentiCRISPRv2 vector for 2 days, and only the surviving cells were cultured. The JAK1 gene knock-out was confirmed using Western blotting, sequencing (Western blotting - FIG. 3C).

To confirm whether the JAK1 knock-out cells produced as described above had the effect of reducing stem cell senescence, it was confirmed by SA-b-gal staining which is a representative cellular senescence measurement technique. The specific SA-b-gal staining method is the same as the method of Example <1-1 >.

As a result, it was confirmed that inflammatory senescence induced by poly IC in the stem cells was markedly reduced by JAK1 knock-out (FIG. 3A).

### <2-2> Confirmation of inhibition of stem cell survival rate decrease after transplantation according to JAK1 knock-out

The present inventors used BrdU assay to observe the effect of inhibiting the decease of the survival rate of stem cells after transplantation according to JAK-1 knock-out cell treatment. The specific BrdU assay method is the same as the method of Example <1-2>.

As a result, as shown in FIG. 3B, it was confirmed that cell survival rate by cellular senescence induced by poly IC in stem cells was less decreased by JAK1 knock-out.

### <2-3> Confirmation of expression of proteins involved in proliferation and differentiation promotion of stem cell and sternness increase according to JAK1 knock-out using Western blotting

The present inventors have confirmed the expression of proteins involved in proliferation and differentiation promotion of stem cell and stemness increase according to JAK1 knock-out cell treatment using Western blotting. The specific Western blotting method is the same as the method of Example <1-3>.

As a result, the decrease in the expression of the protein associated with senescence (PCNA), stemness (EZH2, BMI-1) and proliferation (survivin, CyclinD1) were less decreased according to JAK1 knock-out. In addition, the increase in expression of TLR3 signaling-related proteins (TLR3, IL6) were less increased. (FIG. 3C).

### <2-4> Confirmation of expression of proteins involved in proliferation and differentiation promotion of stem cell and sternness increase according to JAK1 knock-out using real time PCR

The present inventors have confirmed the expression of proteins involved in proliferation and differentiation promotion of stem cell and stemness increase according to JAK1 knock-out cell treatment using real time PCR. The specific real time PCR method is the same as the method of Example <1-4>.

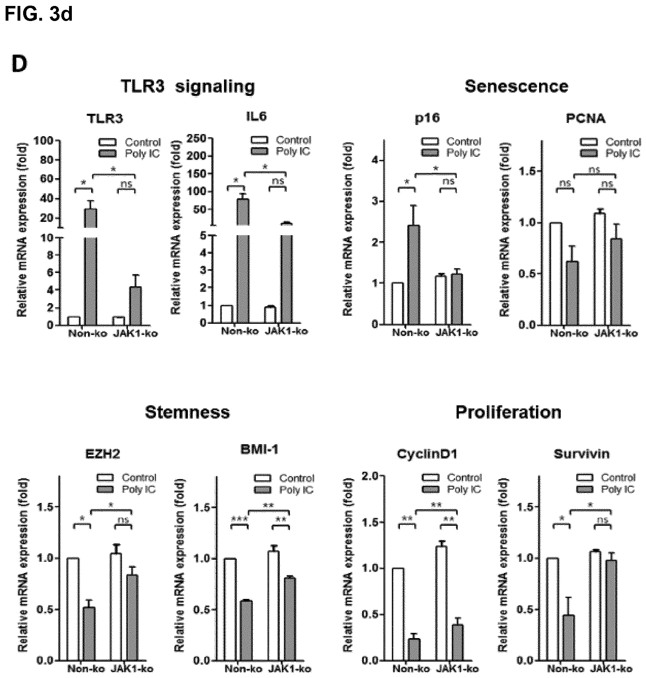

As a result, the decrease in the mRNA expression of the protein associated with senescence (PCNA), stemness (EZH2, BMI-1) and proliferation (survivin, CyclinD1) were less decreased according to JAK1 knock-out. In addition, the increase in expression of TLR3 signaling-related proteins (TLR3, IL6) were less increased. (FIG. 3D).

### <2-5> Confirmation of reduction of immunogenicity by JAK1 knock-out

The present inventors have confirmed the degree of expression of HLA-A and HLA-B which are immunogenicity-related proteins according to JAK1 knock-out cell treatment using FACS analysis and real time PCR. The specific experiment method is the same as the method of Example <1-5>.

As a result, it was confirmed that the immunogenicity-related proteins HLA-A and HLA-B were increased by the poly IC treatment, and this increase was less increased by JAK1 knock-out (FIG. 3E).

### <2-6> Confirmation of increase in immunomodulatory ability by JAK1 knock-out

The present inventors have confirmed the expression level of TGF-b and HGF which are proteins that inhibit T cell activity by JAK1 knock-out cell treatment using real time PCR. The specific experiment method is the same as the method of Example <1-6>.

As a result, it was confirmed that the proteins TGF-b and HGF, which inhibit T cell activation, are reduced by treatment with poly IC, and this decrease is less decreased by JAK1 knock-out (FIG. 3E).

The present invention has been described above with reference to preferred embodiments thereof.

It will be understood by those skilled in the art that various changes in form and details may be made therein without departing from the spirit and scope of the invention as defined by the appended claims. Therefore, the disclosed embodiments should be considered in an illustrative rather than a restrictive sense. The scope of the present invention is defined by the appended claims rather than by the foregoing description, and all differences within the scope of equivalents thereof should be construed as being included in the present invention.

## Claims

1. A composition for proliferation, differentiation promotion or senescence inhibition of stem cells comprising a JAK1 (Janus kinase 1) inhibitor as an active ingredient.

2. The composition for proliferation, differentiation promotion or senescence inhibition of stem cells of claim 1, wherein the JAK1 inhibitor is selected from the group consisting of tofacitinib, baricitinib, ruxolitinib and filgotinib.

3. The composition for proliferation, differentiation promotion or senescence inhibition of stem cells of claim 1, wherein the JAK1 inhibitor inhibits or reduces an expression of HLA-A or HLA-B which is an immunogenicity-related protein, and promotes or increases an expression of TGF-b or HGF which inhibits activity of T cells.

4. The composition for proliferation, differentiation promotion or senescence inhibition of stem cells of claim 1, wherein the JAK1 inhibitor reduces the senescence of stem cells and promotes survival rate of stem cells.

5. A method of promoting proliferation and differentiation of stem cells, comprising: inhibiting an expression or activity of JAK1 (Janus kinase 1) in stem cells.

6. The method of promoting proliferation and differentiation of stem cells of claim 5, wherein the expression or activity of JAK1 (Janus kinase 1) is inhibited by treating a JAK1 (Janus kinase 1) inhibitor or knocking out a JAK1 (Janus kinase 1) gene in a stem cell.

7. The method of promoting proliferation and differentiation of stem cells of claim 6, wherein the JAK1 inhibitor is selected from the group consisting of tofacitinib, baricitinib, ruxolitinib and filgotinib.

8. A method of inhibiting senescence of a stem cell comprising: treating a JAK1 (Janus kinase 1) inhibitor or knocking out a JAK1 (Janus kinase 1) in a stem cell.

9. The method of inhibiting senescence of a stem cell of claim 8, wherein the JAK1 inhibitor is selected from the group consisting of tofacitinib, baricitinib, ruxolitinib and filgotinib.

10. A method of preparing stem cells for cell therapy, comprising treating a JAK1 (Janus kinase 1) inhibitor or knocking out a JAK1 (Janus kinase 1) in a stem cell for cell transplantation, wherein the stem cells have followings:
(a) reduced inflammatory senescence of stem cells;
(b) inhibited cell survival rate reduction of stem cells;
(c) increased cell proliferation of stem cells;
(d) increased stemness of stem cells;
(e) decreased immunogenicity of stem cells; and
(f) increased immunomodulatory activity of stem cells.

11. A stem cell for cell therapy which has followings by treating a JAK1 (Janus kinase 1) inhibitor or knocking out a JAK1 (Janus kinase 1) in a stem cell:
(a) reduced inflammatory senescence of stem cells;
(b) inhibited cell survival rate reduction of stem cells;
(c) increased cell proliferation of stem cells;
(d) increased stemness of stem cells;
(e) decreased immunogenicity of stem cells; and
(f) increased immunomodulatory activity of stem cells.

12. A cell therapeutic composition for preventing or treating immune diseases, comprising the stem cell of claim 11 as an active ingredient.

13. A cell therapy kit for preventing or treating immune diseases, comprising JAK1 (Janus kinase 1) inhibitor and stem cells.

14. A cell therapeutic composition for preventing or treating immune diseases, comprising JAK1 inhibitor and stem cells, as an active ingredient.

15. A method of preventing or treating immune diseases by administering JAK1 inhibitor and stem cells to a subject.
